# EUROPÄISCHE PATENTSCHRIFT

(11) **EP 2 515 812 B1**
(45) Veröffentlichungstag und Bekanntmachung des Hinweises auf die Patenterteilung: **08.05.2013**
(21) Anmeldenummer: 11754831.3
(22) Anmeldetag: 18.08.2011
(51) Int. Cl.: A61F 13/02, A61L 15/26, A61L 15/42, A61M 27/00, A61M 1/00

(54) **VERWENDUNG EINES POLYURETHANSCHAUMSTOFFS ALS WUNDAUFLAGE IN DER UNTERDRUCKTHERAPIE**
USE OF POLYURETHAN FOAM IN NEGATIVE PRESSURE WOUND TREATMENT
UTILISATION DE MOUSSE POLYURÉTHANE POUR LE TRAITEMENT DE PLAIES PAR DÉPRESSION

(30) Priorität: 19.08.2010 DE 102010034819
(43) Veröffentlichungstag der Anmeldung: 31.10.2012
(60) Teilanmeldung: 12008496.7
(73) Patentinhaber: Paul Hartmann AG, 89522 Heidenheim (DE)
(72) Erfinder: ECKSTEIN, Axel, 89522 Heidenheim (DE); CROIZAT, Pierre, 89542 Herbrechtingen (DE); FINK, Ulrich, 89522 Heidenheim (DE); MALOWANIEC, Krzysztof, Daniel, 89522 Heidenheim (DE)
(74) Vertreter: Aechter, Bernd
(86) Internationale Anmeldenummer: PCT/EP2011/004164
(87) Internationale Veröffentlichungsnummer: WO 2012/022485

(56) Entgegenhaltungen:
- WO-A1-01/85248
- WO-A1-93/09727
- WO-A1-2009/126102
- WO-A1-2011/049522
- US-A- 3 572 340
- US-A- 6 071 267

## Beschreibung

Die Erfindung betrifft eine Vorrichtung zur Unterdrucktherapie von Wunden umfassend, (a) ein Abdeckmaterial zum luftdichten Verschließen eines Wundraums; (b) gegebenenfalls ein Mittel zum Anschließen einer Unterdruckquelle; und (c) eine Wundauflage, enthaltend einen offenzelligen Polyurethanschaumstoff, wobei der offenzellige Polyurethanschaumstoff spezielle Eigenschaften, insbesondere eine Zugfestigkeit nach dreitägiger Lagerung in Rinderserum, gemessen gemäß DIN 53571, zwischen 80 kPa und 300 kPa aufweist. Ferner betrifft die Erfindung die Verwendung eines solchen offenzelligen Polyurethanschaumstoffs zur Anwendung als Wundauflage in der Unterdrucktherapie von Wunden.

Unter einer Wunde wird die Trennung des Zusammenhangs von Geweben der Körperhülle bei Menschen oder Tieren verstanden. Sie kann mit einem Verlust an Substanz verbunden sein.

Vorrichtungen zur Unterdrucktherapie von Wunden sind im Stand der Technik bekannt. So beschreibt beispielsweise die WO 1993/009727 A1 eine Vorrichtung zur Förderung der Wundheilung durch die Applikation von Unterdruck auf dem die Wunde aufweisenden und die Wunde umgebenden Hautbereich. Die Vorrichtung gemäß WO 1993/009727 A1 umfasst eine Vakuumeinrichtung zur Erzeugung des Unterdrucks, eine luftdichte Abdeckung der Wunde, welche mit der Vakuumeinrichtung in einer funktionellen Verbindung steht, sowie eine Wundauflage zur Positionierung an der Wunde innerhalb der luftdichten Abdeckung.

Vorrichtungen zur Unterdruchtherapie sind auch in den Druchschriften WO 2009/126102, WO 2011/049522 und US 6 071 267 offenbart.

Geräte zur Unterdrucktherapie von Wunden sind kommerziell erhältlich, beispielsweise das V.A.C.^{®}-Gerät der Firma KCI. Bei kommerziell erhältlichen Geräten wird oftmals eine Wundauflage eingesetzt, welche einen offenzelligen Polymer-Schaumstoff, wie beispielsweise Polyvinylalkohol (PVA) oder Polyurethan (PU), enthält.

Die kommerziell üblichen Schaumauflagen werden, abhängig vom angelegten Unterdruck, unterschiedlich stark komprimiert. Dadurch kann eine Verengung der für den Abtransport des Wundexsudats nötigen Durchgänge entstehen. Weiterhin kann eine Verklebung des Schaums mit dem Wundgrund auftreten. Neu gebildetes Gewebe kann in den Schaum einwachsen. Dieses Problem ist eine bekannte Komplikation bei der Unterdrucktherapie von Wunden (FDA complaint data base). Um dieses Problem zu lösen, werden häufig zusätzliche Wundkontaktschichten zwischen Schaum und Wundgrund eingebracht, beispielsweise eine Folie (siehe z.B. W02001/85248). Diese zusätzlichen Wundkontaktschichten vermindern aber den Durchfluss von Exsudat.

Verklebter Schaum muss zudem beim Wechseln des Verbands aufwendig entfernt werden, beispielsweise durch Spülen mit Ringerlösung. In den Schaum eingewachsenes Gewebe kann beim Verbandwechsel zu einer Gewebetraumatisierung und damit zur Störung der Wundheilung führen.

Beim Einsatz herkömmlicher Wundauflagen können zudem Schaumstoffpartikel in die Wunde gelangen. Diese können zu einer Reizung der Wunde und zu einer verzögerten Wundheilung führen. Dieses Problem wird noch verstärkt, wenn die Wundauflage vor der Anwendung auf die Größe der Wunde zugeschnitten wird, da dann insbesondere lose Schaumstoffpartikel an den Schnitträndern erzeugt werden.

Der vorliegenden Erfindung liegt die Aufgabe zugrunde, die Unterdruckbehandlung von Wunden weiter zu verbessern und die Nachteile des Standes der Technik zu überwinden. Es sollen mit der vorliegenden Erfindung Vorrichtungen und Verfahren zur Unterdruckbehandlung von Wunden zur Verfügung gestellt werden, mit denen eine Behandlung möglichst wirksam und möglichst schonend durchgeführt werden kann. Insbesondere soll die vorliegende Erfindung das Ablagern von Schaumstoffpartikel in der Wunde verhindern.

Die Aufgaben konnten unerwartet durch die Verwendung einer Wundauflage aus einem speziellen Polyurethanschaumstoff gelöst werden. Hierbei wurde insbesondere gefunden, dass die Lagerung des Polyurethanschaumstoffs in Rinderserum die Gegebenheiten bei der Unterdrucktherapie zu simulieren vermag. Sofern der Polyurethanschaumstoff so ausgewählt wird, dass er nach dreitägiger Lagerung in Rinderserum eine vorteilhafte Zugfestigkeit aufweist, führt dies zu einer überraschend starken Verringerung von unerwünschten Schaumstoffpartikeln in der Wunde. Des Weiteren wurde gefunden, dass durch bestimmte weitere physikalische Parameter sowie durch die geeignete Wahl der Ausgangskomponenten Polyurethanschaumstoffe erhältlich sind, welche die vorstehend genannten Aufgaben unerwartet vorteilhaft lösen.

Gegenstand eines ersten Aspekts der Erfindung ist daher eine Vorrichtung zur Unterdrucktherapie von Wunden, wie in Anspruch 1 definiert.

Gegenstand eines zweiten Aspekts der Erfindung ist daher eine Vorrichtung zur Unterdrucktherapie von Wunden, wie in Anspruch 6 definiert.

Gegenstand eines dritten Aspekts der Erfindung ist daher eine Vorrichtung zur Unterdrucktherapie von Wunden, umfassend
(a) ein Abdeckmaterial zum luftdichten Verschließen eines Wundraums;
(b) gegebenenfalls ein Mittel zum Anschließen einer Unterdruckquelle; und
(c) einen offenzelligen Polyurethanschaumstoff als Wundauflage, erhältlich durch Umsetzung einer Mischung, umfassend die Komponenten
   (i) Polyisocyanat, ausgewählt aus MDI, PMDI, TDI und/oder HDI,
   (ii) Polyesterpolyol, wobei das Polyesterpolyol bevorzugt erhältlich ist durch Umsetzung einer Dicarbonsäure mit 4 bis 8 Kohlenstoffatomen mit einen Dialkohol mit 2 bis 6 Kohlenstoffatomen und/oder bevorzugt ein gewichtsmittleres Molekulargewicht von 500 bis 4000 g/mol aufweist,
   (iii) Treibmittel, und
   (iv) Katalysator.

Ebenfalls umfasst die Erfindung beliebige Kombinationen der genannten Aspekte. Ferner ist Gegenstand der Erfindung die Verwendung der vorstehend in den drei Aspekten beschriebenen offenzelligen Polyurethanschaumstoffe (c), z.B. die Verwendung eines offenzelligen Polyurethanschaumstoffs, der eine Zugfestigkeit nach dreitägiger Lagerung in Rinderserum, gemessen gemäß DIN 53571, zwischen 80 kPa und 300 kPa aufweist, als Wundauflage zur oder in der Unterdrucktherapie von Wunden.

Die neue erfindungsgemäße Vorrichtung bzw. die erfindungsgemäße Verwendung der Wundauflage zeichnet sich durch mehrere unerwartete Vorteile aus.

Insbesondere konnte durch die Wahl der Eigenschaften des Schaumstoffs die Anzahl der Partikel, welche unerwünscht in die Wunde gelangen, vorteilhaft reduziert werden.

Die Verwendung der erfindungsgemäßen Wundauflage verbesserte zudem die atraumatischen Eigenschaften, so dass eine Unterdruckbehandlung auch ohne zusätzliche Wundkontaktschichten ermöglicht wurde.

Die erfindungsgemäße Wundauflage ermöglicht eine ausreichende Durchleitung von Wundexsudat, fühlt sich verhältnismäßig angenehm an und trägt somit zu einer Erhöhung der Patientencompliance (Befolgung der Therapieanweisung durch den Patienten) bei.

Die Bestandteile (a) bis (c) der erfindungsgemäßen Vorrichtung werden nachstehend beschrieben.

Die erfindungsgemäße Vorrichtung umfasst ein Abdeckmaterial (a) zum luftdichten Verschließen des Wundraums. Unter Wundraum wird die Wunde und gegebenenfalls die anliegende Wundumgebung verstanden. Unter "luftdichtem Verschließen" soll hier nicht verstanden werden, dass keinerlei Gasaustausch zwischen dem Wundraum und seiner Umgebung stattfindet. Vielmehr ist mit "luftdichtem Verschließen" in diesem Zusammenhang gemeint, dass unter Berücksichtigung der eingesetzten Unterdruckpumpe der für die Unterdrucktherapie von Wunden nötige Unterdruck aufrecht erhalten werden kann. Es können deshalb auch Abdeckmaterialien eingesetzt werden, welche eine geringfügige Gaspermeabilität aufweisen, solange der für die Unterdrucktherapie notwendige Unterdruck aufrechterhalten werden kann.

In einer bevorzugten Ausführungsform der Erfindung umfasst das Abdeckmaterial zum luftdichten Verschließen der Wunde ein wasserunlösliches Polymer oder eine Metallfolie. Das Abdeckmaterial weist bevorzugt eine Dicke von 10 µm bis 10.000 µm, insbesondere von 25 µm bis 100 µm, auf.

In einer bevorzugten Ausführungsform der Erfindung handelt es sich bei dem Abdeckmaterial (a) um ein wasserunlösliches Polymer. Bevorzugt weist das wasserunlösliche Polymer eine Löslichkeit von 10 mg/l oder weniger, mehr bevorzugt von 1 mg/ml oder weniger, insbesondere von 0,0001 bis 1 mg/ml auf (bestimmt gemäß Säulenelutionsmethode nach EU-Richtlinie RL67-548-EWG, Anhang V Kap. A6). Beispiele sind Polyurethan, Polyester, Polypropylen, Polyethylen, Polyamid oder Polyvinylchlorid, Polyorganosiloxan (Silikon) oder eine Mischung daraus. Die genannten Polymere liegen hierbei bevorzugt in nicht-zellulärer Form vor.

Es hat sich gezeigt, dass durch ein Abdeckmaterial mit einer speziellen Wasserdampfdurchlässigkeit die eingangs erläuterten Aufgaben besonders vorteilhaft gelöst werden können. In einer bevorzugten Ausführungsform weist daher das Abdeckungsmaterial einen Wasserdampfdurchlässigkeit von 100 bis 2500 g/m² x 24h, mehr bevorzugt von 500 bis 2000 g/m² x 24h, noch mehr bevorzugt von 800 bis 1600 g/m² x 24h, insbesondere von 1050 bis 1450 g/m² x 24h, bestimmt gemäß DIN EN 13726-2 bei 23 °C und 85 % relativer Luftfeuchte, auf. Insbesondere die Kombination einer Abdeckfolie (a) mit vorstehend genannter Wasserdampfdurchlässigkeit mit einem offenzelligen Polyurethanschaumstoff mit nachstehend beschriebenen physikalischen Eigenschaften ist besonders vorteilhaft.

Die erfindungsgemäße Vorrichtung zur Unterdrucktherapie umfasst gegebenenfalls ein Mittel (b) zum Anschließen einer Unterdruckquelle, d.h. ein Mittel zur Herstellung von Unterdruck im Wundraum. In einer bevorzugten Ausführungsform handelt es sich hierbei um ein Mittel (b) zur funktionellen Verbindung des Wundraums mit einer außerhalb des Abdeckmaterials befindlichen Unterdruckquelle, so dass ein Unterdruck im Wundraum herstellbar ist und Flüssigkeiten aus dem Wundraum absaugbar sind.

Der Ausdruck "Unterdruck im Wundraum" bezeichnet im Zusammenhang mit der Erfindung einen innerhalb des Wundverbandes gegenüber dem Umgebungsluftdruck (atmosphärischer Luftdruck) erniedrigten Luftdruck. Unter "innerhalb des Wundverbandes" wird der durch das Abdeckmaterial und die Wunde gebildete Zwischenraum verstanden.

Die Druckdifferenz zwischen dem Luftdruck innerhalb des Wundverbandes und dem Umgebungsluftdruck wird im Zusammenhang mit der Erfindung in mm Hg (Millimeter-Quecksilbersäule) angegeben, da dies im Bereich der Unterdrucktherapie üblich ist. 1 mm Hg entspricht einem Torr beziehungsweise 133,322 Pa (Pascal). Im Zusammenhang mit der Erfindung wird der Unterdruck, d. h. die Druckdifferenz zwischen dem Luftdruck innerhalb des Wundverbandes und dem Umgebungsluftdruck, als positiver Zahlenwert in mm Hg angegeben.

In einer Ausführungsform der Erfindung handelt es sich bei dem Unterdruck um einen Unterdruck von mindestes 25 mm Hg und höchstens 250 mm Hg, bevorzugt von mindestens 50 mm Hg und höchstens 150 mm Hg. Dieser Unterdruckbereich hat sich als für die Wundheilung vorteilhaft erwiesen. In einer bevorzugten Ausführungsform der Erfindung handelt es sich bei dem Unterdruck um einen Unterdruck von mindestens 80 mm Hg und höchstens 140 mm Hg, mehr bevorzugt von mindestens 120 mm Hg und höchstens 130 mm Hg.

Die erfindungsgemäße Vorrichtung zur Unterdrucktherapie von Wunden umfasst, wie vorstehend geschildert, bevorzugt ein Mittel (b) zum Anschließen einer Unterdruckquelle, d.h. ein Mittel zur funktionellen Verbindung des Wundraums mit einer außerhalb des Abdeckmaterials befindlichen Unterdruckquelle.

Die funktionelle Verbindung kann beispielsweise mit einer Verbindungsleitung oder mit einem Unterdruck-Anschlussstück hergestellt werden. Unterdruck-Anschlussstücke sind dem Fachmann auch unter der Bezeichnung "Port" bekannt.

Bei einer Ausführungsform handelt es sich bei dem Mittel (b) um eine Verbindungsleitung, bevorzugt ein Schlauch, insbesondere ein Silikon-Drainageschlauch. Die Verbindungsleitung kann durch das Abdeckmaterial hindurchgeführt werden. Alternativ kann die mindestens eine Verbindungsleitung unter dem Rand des Abdeckmaterials durchgeführt werden. In beiden Fällen ist die Durchtrittsstelle luftdicht abzudichten, sodass der gewünschte Unterdruck im Verband aufrechterhalten werden kann.

In einer weiteren bevorzugten Ausführungsform handelt es sich bei dem Mittel (b) um ein Unterdruck-Anschlussstück (Port), welches auf einer der Innen- oder Außenseiten des Abdeckmaterials befestigt werden kann, wobei das Abdeckmaterial geeignete Öffnungen aufweist. Auch bei dieser Ausführungsform ist auf eine luftdichte Abdichtung entweder der Durchtrittsöffnung (Port innen) oder der Auflagefläche (Port außen) zu achten. Die Abdichtung kann beispielsweise mit einer Klebefolie, mit einer Klebemasse, oder mit einem Klebestreifen hergestellt werden.

Neben den vorstehend beschriebenen Bestandteilen (a) und optional (b) umfasst die erfindungsgemäße Vorrichtung noch Bestandteil (c). Die Wundauflage (c), welche in der erfindungsgemäßen Vorrichtung Verwendung findet, wird nachstehend genauer beschrieben. Alle Erläuterungen zur Wundauflage (c) betreffen nicht nur die erfindungsgemäße Vorrichtung, sondern auch das erfindungsgemäße Verfahren zur Herstellung der Wundauflage und die erfindungsgemäße Verwendung der Wundauflage in der Unterdrucktherapie von Wunden.

Die Wundauflage (c) enthält einen offenzelligen Polyurethanschaumstoff (PUR-Schaumstoff). Als Schaumstoffe werden üblicherweise Werkstoffe mit über die gesamte Masse verteilten Zellen (offen, geschlossen oder beides) verstanden. Solche Werkstoffe weisen üblicherweise somit eine Rohdichte (gemäß DIN EN ISO 845) auf, die niedriger ist als die Dichte der Gerüstsubstanz.

Eine Zelle ist der bei der Herstellung von Schaumstoffen gebildete einzelne Hohlraum, der von Zellwänden und/oder Zellstegen teilweise oder vollständig umschlossen ist.

Eine geschlossene Zelle ist üblicherweise eine Zelle, die vollständig von ihren Wänden umschlossen ist und daher nicht mit anderen Zellen über die Gasphase in Verbindung steht. Eine offene Zelle ist üblicherweise eine Zelle, die über die Gasphase mit anderen Zellen in Verbindung steht. Im Rahmen dieser Anmeldung bedeutet der Begriff offenzellig, dass im Polyurethanschaumstoff mindestens 60 % offene Zellen, bevorzugt mindestens 90 % offene Zellen, mehr bevorzugt mindestens 98 % offene Zellen, insbesondere im Wesentlichen 100 % offene Zellen, bezogen auf die Gesamtzahl an Zellen, vorhanden sind. Die Offenzelligkeit des Polyurethanschaumstoffs wird üblicherweise nach ASTM D 2856-87, Verfahren B) bestimmt.

Unter Zellwand wird üblicherweise die die Zelle umschließende Wand verstanden. Die Zellwand kann auch als Zellmembran bezeichnet werden. Als Zellsteg wird üblicherweise der Bereich der Zellwand verstanden, der mehr als zwei Zellen voneinander trennt. Zellstege weisen bevorzugt mindestens das 1,5-fache der Dicke, mehr bevorzugt mindestens das 2-fache der Dicke, des übrigen Bereichs der Zellwand auf.

Bei dem offenzelligen Polyurethanschaumstoff kann es sich um einen retikulierten oder um einen nicht-retikulierten Schaumstoff handeln. Unter einem retikulierten Schaumstoff wird ein Schaumstoff verstanden, der im Wesentlichen nur Zellstege aufweist. Bei einem retikulierten Schaumstoff sind die Zellwände somit im Wesentlichen entfernt. Die Retikulierung wird üblicherweise in einer Druckkammer, z.B. einer Stahlkammer durchgeführt. Nach Einbringen des Schaumstoffs in die Stahlkammer wird die Luft abgesaugt (bevorzugt zu 50 bis 100 Gew.-%, mehr bevorzugt zu 70 bis 99 Gew.-%) und durch ein Brenngasgemisch, bevorzugt durch ein Gemisch enthaltend Wasserstoff und Sauerstoff, insbesondere im molaren Verhältnis von 2 : 1 ersetzt. Bei der Zündung des Gasgemisches zerreißen die Zellhäute durch die entstehende Hitze- und Druckwelle. Gegebenenfalls erfolgt auch ein zumindest teilweises Aufschmelzen der Zellstege, so dass diese verstärkt werden.

Der Schaumstoff (cl) weist üblicherweise eine Zellzahl (= Anzahl der Poren entlang einer Geraden pro laufendem cm) von 3 bis 40 cm⁻¹, bevorzugt 5 bis 25 cm⁻¹, mehr bevorzugt 7 bis 18 cm⁻¹, noch mehr bevorzugt 8 bis 15 cm⁻¹, auf. Die Zellzahl wird bevorzugt mikroskopisch bestimmt.

Grundsätzlich sollte der offenzellige Polyurethanschaumstoff bestimmten physikalischen Anforderungen genügen. Es hat sich nämlich gezeigt, dass vorstehend genannte Aufgaben unerwartet vorteilhaft gelöst werden können, wenn der Polyurethanschaumstoff eine spezielle Zugfestigkeit, eine spezielle Bruchdehnung und/oder einer spezielle Härte aufweist.

Gemäß der Erfindung weist der Polyurethanschaumstoff üblicherweise eine Zugfestigkeit nach dreitägiger Lagerung in Rinderserum zwischen 80 kPa und 300 kPa, bevorzugt zwischen 110 kPa bis 250 kPa, mehr bevorzugt zwischen 120 kPa bis 230 kPa, noch mehr bevorzugt von 130 bis 220 kPa, besonders bevorzugt von 140 bis 200 kPa, ganz besonders bevorzugt von 155 bis 190 kPa und insbesondere von 160 bis 185 kPa, auf.

Rinderserum ist an sich im Fachgebiet bekannt. Hierbei handelt es sich um ein Serum, das von Rinderblut gewonnen wird. Bevorzugt wird das unter dem Handelsnamen HyClone^{®} "Standard Fetal Bovine Serum" vertriebene Rinderserum der Firma Thermo Scientific verwendet. In einer bevorzugten Ausführungsform weist das verwendete Rinderserum im Wesentlichen folgende Zusammensetzung bzw. Eigenschaften auf:

| **Proteingehalt und weitere Werte** | | |
|---|---|---|
| Albumin | 1,9 | gm/dl |
| Alkalische Phosphatase | 213 | mU/ml |
| Blut-Harnstoff Stickstoff | 12 | mg/dl |
| Kreatinin | 2,77 | mg/dl |
| Gammaglobulin | 1,7 | % tp |
| Blutzucker (Glucose) | 107 | mg/dl |
| Glutamat-Oxalacetat-Transaminase (SGOT) | 152 | mU/ml |
| Glutamat-Pyruvat-Transaminase (SGPT) | 37 | mU/ml |
| IgG - Nephelometer | 0,14 | mg/ml |
| Lactatdehydrogenase | 2479 | mU/ml |
| Osmolalität | 312 | mOsm/kg |
| pH | 7,18 | |
| Gesamtbilirubin | 0,4 | mg/dl |
| Gesamtprotein | 3,7 | gm/dl |

| **Gehalt Spurenelemente und Eisen** | | |
|---|---|---|
| Calcium | 13,1 | mg/dl |
| Chlorid | 99 | mEq/l |
| Anorganischer Phosphor | 9,6 | mg/dl |
| Eisen | 160 | ug/dl |
| Sättigungskonzentration (Eisen) | 79 | % |
| Kalium | >10,0 | mEq/l |
| Natrium | 133 | mEq/l |
| Gesamt-Eisenbindungskapazität (TIBC) | 201 | ug/dl |

Die zu vermessende Probe wird für 3 Tage bei 23 °C in Rinderserum eingelegt und untergetaucht. Anschließend wird die Zugfestigkeit gemäß DIN 53571 bestimmt. Im Rahmen dieser Anmeldung bedeutet der Ausdruck "gemäß DIN 53571", dass die Bestimmung der Zugfestigkeit grundsätzlich gemäß dieser Norm erfolgt, wobei jedoch in Abweichung zur Norm die 3 Tage in Rinderserum gelagerte Probe nicht vollständig getrocknet wird. Stattdessen wird die Probe aus dem Rinderserum entnommen und in 1 Liter Wasser zum Spülen getaucht. Anschließend wird der Probekörper mit Zellstoffpapier ausgedrückt. Zudem wird in Abweichung zur Norm ein rechteckiger Probekörper mit den Abmessungen 10 x 12,5 x 75 verwendet.

Die Messung der Zugfestigkeit erfolgt mit einem Zugprüfgerät nach EN ISO 527-1 [April 1996] der Firma Zwick (Ulm). Folgende Prüfparameter werden verwendet:

| | |
|---|---|
| Prüfgeschwindigkeit: | 500 mm/min |
| Einspannlänge: | 50 mm |
| Vorkraft: | 0,1 N |
| Probenbreite b0: | 12,5 mm. |

Ferner weist der Polyurethanschaumstoff (c) bevorzugt eine Bruchdehnung von 150 % bis 700 %, mehr bevorzugt von 200 % bis 650 %, noch mehr bevorzugt von 240 % bis 340 %, insbesondere 260 bis 320 %, gemessen gemäß DIN 53571 (Verfahren 1, Probekörper A), auf. Zudem weist der Polyurethanschaumstoff bevorzugt eine Härte von 20 bis 70 Shore A, mehr bevorzugt von 30 bis 60 Shore A, noch mehr bevorzugt von 40 bis 50 Shore A, gemessen gemäß DIN 53505, auf, wobei die Messung bei 23 °C an einem plattenförmigen, ebenen und glatten Probekörper einer Dicke von 6 mm erfolgte.

Es hat sich ferner gezeigt, dass vorstehend genannte Aufgaben unerwartet vorteilhaft gelöst werden können, wenn der Polyurethanschaumstoff eine spezielle Luftdurchlässigkeit aufweist. In einer bevorzugten Ausführungsform weist der Polyurethanschaumstoff eine Luftdurchlässigkeit von 1000 bis 8000 1/(m²sec), mehr bevorzugt von 1500 bis 6000 l/(m²sec), noch mehr bevorzugt von 2000 bis 5000 1/(m²sec), besonders bevorzugt von 2300 bis 4000 1/(m²sec) und insbesondere von 2400 bis 3300 l/(m²sec), gemessen gemäß DIN EN ISO 9237 (20 mm Prüfdicke, 20 cm² Prüffläche, 200 Pa Differenzdruck) auf.

Es hat sich weiterhin gezeigt, dass vorstehend genannte Aufgaben unerwartet vorteilhaft gelöst werden können, wenn der Polyurethanschaumstoff viskoelastisches Verhalten zeigt. Hierunter wird verstanden, dass das Verhalten des Polyurethanschaumstoffs auf Beanspruchung so aussieht, wie die Kombination aus einem elastischen Feststoff und einer viskosen Flüssigkeit. Das viskoelastische Verhalten kann durch einen Torsionsschwingungsversuch gemäß DIN 53445, Verfahren A, charakterisiert werden. Es ist bevorzugt, dass der Schaumstoff bei der Bestimmung gemäß DIN 53445, Verfahren A bei 23 °C einen mechanischen Verlustfaktor von 0,1 bis 1,0, mehr bevorzugt von 0,15 bis 0,8, noch mehr bevorzugt von 0,2 bis 0,6, aufweist.

Es hat sich weiterhin gezeigt, dass vorstehend genannte Aufgaben unerwartet vorteilhaft gelöst werden können, wenn der Schaumstoff (c) eine Rohdichte zwischen 15 und 55 kg/m³, mehr bevorzugt zwischen 20 und 35 kg/m³, noch mehr bevorzugt zwischen 22 und 30 kg/m³, insbesondere zwischen 24 und 28 kg/m³, aufweist, gemessen gemäß DIN EN ISO 845 (Probekörper mit Abmessungen 100 mm x 100 mm x 50 mm, Konditionierung für 24 h im Normklima (23 °C, 50 % rel. Luftfeuchte, 1013 mbar)).

Sofern in den einschlägigen Normen nichts anderes angegeben, werden im Allgemeinen alle Testmethoden bei 23 °C, 50 % rel. Luftfeuchte und 1013 mbar Druck durchgeführt.

Bevorzugte Ausführungsformen der einsetzbaren Polyurethanschaumstoffe (c-PUR) werden nachstehend erläutert. Der Polyurethanschaumstoff ist üblicherweise erhältlich durch Umsetzung einer Mischung, umfassend die Komponenten
(i-PUR) Polyisocyanat,
(ii-PUR) gegenüber Isocyanat reaktive Verbindungen, insbesondere Polyol,
(iii-PUR), Katalysator,
(iv-PUR) Treibmittel, und
(v-PUR) gegebenenfalls Zusatzstoffe.

Als Isocyanate (i-PUR) können allgemein bekannte aliphatische, cycloaliphatische und/oder insbesondere aromatische Polyisocyanate eingesetzt werden. Zur Herstellung der Polyurethane eignen sich beispielsweise Diphenylmethandiisocyanat (MDI), hier insbesondere 4,4'- Diphenylmethandiisocyanat (4,4'-MDI), Mischungen aus monomeren Diphenylmethandiisocyanaten und höherkernigen Homologen des Diphenylmethandiisocyanats (PMDI), Tetramethylendiisocyanat (TMDI), Hexamethylendiisocyanat (HDI), Toluylendiisocyanat (TDI) oder Mischungen daraus.

Bevorzugt wird MDI, insbesondere 4,4'-MDI und/oder HDI verwendet. Das besonders bevorzugt verwendete 4,4'-MDI kann geringe Mengen, bis etwa 10 Gew.-%, allophanat-oder uretonimin-modifizierte Polyisocyanate enthalten. Es können auch geringe Mengen Polyphenylenpolymethylenpolyisocyanat (PMDI) eingesetzt werden. Die Gesamtmenge dieser PMDI sollte 5 Gew.-% des eingesetzten Isocyanats nicht überschreiten.

Die Polyisocyanatkomponente (i-PUR) wird bevorzugt in Form von Polyisocyanatprepolymere eingesetzt. Diese Polyisocyanatprepolymere sind erhältlich, indem vorstehend beschriebene Polyisocyanate (i-PUR), beispielsweise bei Temperaturen von 30 bis 100 °C, bevorzugt bei etwa 80 °C mit einem Unterschuss an nachstehend beschriebenen Polyolen (ii-PUR) zum Prepolymer umgesetzt werden. Das Polyol-Polyisocyanat-Verhältnis wird hierbei so gewählt, dass der NCO-Gehalt des Prepolymeren 8 bis 28 Gew. -%, vorzugsweise 14 bis 26 Gew. -%, besonders bevorzugt 17 bis 23 Gew. -%, beträgt.

Als gegenüber Isocyanaten reaktive Verbindungen (ii-PUR) werden Polyesterole verwendet.

verwendeten Polyesterole (ii-PUR) werden im allgemeinen durch Kondensation von Diolen, mit 2 bis 6 Kohlenstoffatomen, mit Di Carbonsäuren mit 4 bis 8 Kohlenstoffatomen hergestellt. Beispiele für geeignete Säuren sind Bernsteinsäure, Glutarsäure, Adipinsäure, Phthalsäure, Isophthalsäure, und/oder Terephthalsäure und Gemischen hiervon, hergestellt. Adipinsäure ist besonders bevorzugt. Beispiele für geeignete zwei- und mehrwertige Alkohole sind Ethandiol, Diethylenglykol, 1,4-Butandiol, 1,5-Pentandiol, und/oder 1,6-Hexandiol und Gemische hiervon. 1,4-Butandiol ist besonders bevorzugt.

Die Umsetzungsbedingungen von Carbonsäure und Alkohol werden üblicherweise so gewählt, dass die resultierenden Polyesterole keine freien Säuregruppen aufweisen. Ferner weisen die resultierenden Polyesterole im allgemeinen ein gewichtsmittleres Molekulargewicht (bestimmt mittels Gelpermeationschromatographie) von 500 bis 3500 g/mol, bevorzugt von mehr als 1000 g/mol bis 3000 g/mol, insbesondere von 1500 bis 2500 g/mol, auf. Im allgemeinen weisen die eingesetzten Polyesterole eine mittlere theoretische Funktionalität von 2,0 bis 4, gegebenenfalls von mehr als 2 bis weniger als 3, auf. Des Weiteren weisen im allgemeinen die eingesetzten Polyesterole eine mittlere OH-Zahl von 20 bis 150, bevorzugt von 30 bis 80, auf.

In einer bevorzugten Ausführungsform weisen die verwendeten Polyesterole eine Viskosität von 150 mPa·s bis 600 mPa·s, bevorzugt von 200 mPa·s bis 550 mPa·s, mehr bevorzugt von 220 mPa·s bis 500 mPa·s, besonders bevorzugt von 250 mPa·s bis 450 mPa·s und insbesondere von 270 mPa·s bis 350 mPa·s, gemessen nach DIN 53 015 bei 75 °C, auf.

Die Verbindungen (ii-PUR) können in Mischung mit Kettenverlängerungs- und/oder Vernetzungsmitteln verwendet werden. Bei den Kettenverlängerungsmitteln handelt es sich überwiegend um 2-funktionelle Alkohole mit Molekulargewichten von 60 bis 499, beispielsweise Ethylenglykol, Propylenglykol, Butandiol-1,4. Pentandiol-1,5, Dipropylenglykol und/oder Tripropylenglykol. Bei den Vernetzungsmitteln handelt es sich um Verbindungen mit Molekulargewichten von 60 bis 499 und 3 oder mehr aktiven H-Atomen, vorzugsweise Aminen, und besonders bevorzugt Alkoholen, beispielsweise Glyzerin, Trimethylolpropan und/oder Pentaerythrit.

In einer bevorzugten Ausführungsform enthält (oder besteht aus) die Komponente (ii-PUR) 0-25 Gew.-%, bevorzugt 1 bis 20 Gew.-%, Kettenverlängerungs- und/oder Vernetzungsmittel und 75 bis 100 Gew.-%, bevorzugt 80 bis 99 Gew.-% Polyole(n), insbesondere Polyesterpolyole(n), bezogen auf das Gesamtgewicht der Komponente (ii-PUR)

Als Katalysatoren (iii-PUR) können Verbindungen eingesetzt werden, welche die Reaktion der Komponente (i-PUR) mit der Komponente (ii-PUR) beschleunigen. In Frage kommen beispielsweise tertiäre Amine und/oder organische Metallverbindungen, insbesondere Zinnverbindungen. Beispielsweise können als Katalysatoren folgende Verbindungen eingesetzt werden: Triethylendiamin, Aminoalkyl- und/oder Aminophenylimidazole und/oder Zinn-(II)salze von organischen Carbonsäuren. Katalysatoren werden im allgemeinen in einer Menge von 0,1 bis 5 Gew.-% bezogen auf das Gewicht der Komponente (ii-PUR) eingesetzt.

Als Treibmittel (iv-PUR) können allgemein bekannte chemisch oder physikalisch wirkende Verbindungen eingesetzt werden. Als physikalisch wirkendes Treibmittel kann bevorzugt Wasser eingesetzt werden, welches durch Reaktion mit den Isocyanatgruppen Kohlendioxid bildet. Beispiele für physikalische Treibmittel sind (cyclo)aliphatische Kohlenwasserstoffe, vorzugsweise solche mit 4 bis 8, besonders bevorzugt 4 bis 6 und insbesondere 5 Kohlenstoffatomen, teilhalogenierte Kohlenwasserstoffe oder Ether, Ketone oder Acetate. Die Menge der eingesetzten Treibmittel richtet sich nach der angestrebten Dichte der Schaumstoffe. Die unterschiedlichen Treibmittel können einzeln oder in beliebigen Mischungen untereinander zum Einsatz kommen. Besonders bevorzugt wird nur Wasser als Treibmittel eingesetzt, im allgemeinen in einer Menge von 0,1 bis 5 Gew.-%, insbesondere von 2,5 bis 4 Gew.-%, bezogen auf das Gewicht der Komponente (ii-PUR). Physikalische Treibmittel werden bevorzugt in einer Menge von < 0,5 Gew.-%, bezogen auf das Gewicht der Komponente (ii-PUR), eingesetzt.

Die Umsetzung erfolgt gegebenenfalls in Anwesenheit von (v-PUR) Hilfs- und/oder Zusatzstoffen, wie z. B. Füllstoffen, Zellreglern, Zellöffnern, oberflächenaktiven Verbindungen und/oder Stabilisatoren gegen oxidativen, thermischen oder mikrobiellen Abbau oder Alterung.

Zur Herstellung von Polyurethanschaumstoffen werden im allgemeinen die Komponenten (i-PUR) und (ii-PUR) in solchen Mengen zur Umsetzung gebracht, dass das Äquivalenzverhältnis von NCO-Gruppen zur Summe der reaktiven Wasserstoffatome (insbesondere zu Summe der OH-Gruppen) 1 :0,8 bis 1:1,25, vorzugsweise 1 :0,9 bis 1 :1,15, beträgt. Ein Verhältnis von 1:1 entspricht hierbei einem NCO-Index von 100. Die gewünschte Offenzelligkeit des Polyurethanschaums wird im allgemeinen durch eine dem Fachmann bekannte geeignete Wahl der Komponenten (i-PUR) bis (v-PUR) gewährleistet. Gegebenenfalls wird nach der Aushärtung der resultierende PUR-Schaumstoff retikuliert. Hierfür wird auf oben gegebene Erläuterungen verwiesen.

Es hat sich weiterhin gezeigt, dass vorstehend genannte Aufgaben unerwartet vorteilhaft gelöst werden können, wenn der Polyurethanschaumstoff (c) Silber in Form von Silberionen oder in Form von atomarem Silber enthält. Bevorzugt wird nach der Herstellung des Polyurethanschaumstoffs eine Silberbeschichtung aufgebracht. Alternativ kann das Silber bereits in die härtbare Zusammensetzung miteingebracht werden. Bevorzugt enthält der Polyurethanschaumstoff 0,000001 bis 0,1 Gew.-%, mehr bevorzugt 0.0001 bis 0,01 Gew.-% Silber, bezogen auf das Gesamtgewicht des Polyurethanschaumstoffs.

Es hat sich ferner gezeigt, dass die eingangs beschriebenen Aufgaben mit Polyurethanen auf Basis von lediglich aliphatischen Ausgangsstoffen nicht immer befriedigend gelöst werden konnten. Vielmehr wirkt sich die Verwendung von aromatischen Aufbaukomponenten (i-PUR und/oder ii-PUR) vorteilhaft aus. In einer bevorzugten Ausführungsform weist deshalb der Polyurethanschaumstoff (c) einen Aromatengehalt von 5 bis 50 %, mehr bevorzugt von 10 bis 45 %, insbesondere von 15 bis 40 % auf. Der Aromatengehalt wird bestimmt durch das Verhältnis Gewicht an aromatischen Ringen zu Gesamtgewicht des Schaumstoffs.

In einer bevorzugten Ausführungsform der Erfindung weist der offenzellige Polyurethanschaumstoff eine Dicke von 1 bis 50 mm, insbesondere von 15 mm bis 35 mm, auf.

Der offenzellige Polyurethanschaumstoff kann auch in trockenem Zustand mit einer Salbengrundlage, insbesondere einer Triglycerid-Salbengrundlage, benetzt sein. Eine besonders bevorzugte Salbengrundlage enthält:
20 bis 90 Gew.-%, bevorzugt 55 bis 80 Gew.-% Triglyceride, insbesondere enthaltend Fettsäurereste, ausgewählt aus Caprylsäure, Caprinsäure, Laurinsäure und/oder Stearinsäure;
5 bis 75 Gew.-%, bevorzugt 15 bis 45 Gew.-% Diglyceride, insbesondere enthaltend Fettsäurereste, ausgewählt aus Isostearinsäure, Stearinsäure, 12-Hydroxystearinsäure und/oder Adipinsäure; und
0 bis 30 Gew.-%, bevorzugt 5 bis 20 Gew.-% Polyethylenglykol mit einem gewichtsmittleren Molekulargewicht von 500 bis 3000 g/mol. In einer bevorzugten Ausführungsform beträgt der Anteil an Salbengrundlage 10 bis 95 Gew.-%, mehr bevorzugt 30 bis 92 Gew.-%, noch mehr bevorzugt 45 bis 90 Gew.-%, besonders bevorzugt 55 bis 88 Gew.-%, insbesondere 65 bis 85 Gew.-%, bezogen auf das Gesamtgewicht aus Schaumstoff und Salbengrundlage.

Bevorzugt ist der Schaumstoff jedoch z.B. nicht mit einer Aktivierungslösung (wie z.B. Ringerlösung) getränkt. Ebenfalls ist es bevorzugt, dass der offenzellige Polyurethanschaumstoff nicht mit einem Silikongel, z.B. einem hydrophoben Silikongel, beschichtet oder imprägniert ist.

Des Weiteren kann der Polyurethanschaumstoff auch organische Substanzen mit antimikrobieller Wirkung enthalten. Bei der Substanz mit antimikrobieller Wirkung kann es sich beispielsweise um Substanzen mit Amino oder Iminogruppen handeln. Weiterhin kann es sich bei der Substanz mit antimikrobieller Wirkung um antimikrobiell wirksame Metallkationen handeln, insbesondere um Silberkationen, beispielsweise um einen Komplex von 1-Vinyl-2-Pyrrolidone mit Silberkationen. Besonders geeignete Substanzen mit antimikrobieller Wirkung sind weiterhin Biguanid-Derivate wie Chlorhexidin oder Polybiguanide, wie Polyethylenbiguanid (PEB), Polytetramethylenbiguanid (PTMB) oder Polyethylenhexamethylenbiguanid (PEHMB). Ein besonders bevorzugtes Polybiguanid ist Polyhexamethylenbiguanid (PHMB bzw. Polyhexanid). Weitere geeignete Substanzen mit antimikrobieller Wirkung sind Polyguanidine wie zum Beispiel Polyhexamethylenguanidine (PHMG), N-Octyl-1-[10-(4-Octyliminopyridin-1-yl)decyl]pyridin-4-imin (Octenidin), quartäre Ammoniumverbindungen, wie zum Beispiel Benzalkoniumchlorid oder Cetylpyridiniumchlorid, Triazine wie zum Beispiel 1-(3-Chloroallyl)-3,5,7-triaza-1-azonia-adamantan-Chlorid oder die Ammoniumverbindung Taurolidin.

Bevorzugt wird der offenzellige Polyurethanschaumstoff mit den vorstehend erläuterten Substanzen mit antimikrobieller Wirkung imprägniert oder beschichtet.

Substanzen mit antimikrobieller Wirkung sind üblicherweise in dem Polyurethanschaumstoff in einer Menge von 0 bis 30 Gew.-%, bevorzugt von 0.1 bis 15 Gew.-%, bezogen auf das Gesamtgewicht des Polyurethanschaumstoffs, enthalten.

Grundsätzlich gilt, dass alle Erläuterungen zu bevorzugten Ausführungsformen einzelner Parametern des Polyurethanschaumstoffs (c) nicht isoliert zu betrachten sind, sondern auch in Kombination mit den Erläuterungen zu bevorzugten Ausführungsformen anderer Parametern bzw. zu den Stoffzusammensetzungen gelten. So kann es sich bei der erfindungsgemäßen Vorrichtung und bei der erfindungsgemäßen Verwendung beispielsweise um einen offenzelligen Schaumstoff (c) handeln,
der nach dreitägiger Lagerung in Rinderserum eine Zugfestigkeit von 80 kPa bis 300 kPa, bevorzugt von 110 kPa bis 250 kPa, mehr bevorzugt von 120 kPa bis 230 kPa, noch mehr bevorzugt von 130 kPa bis 220 kPa, besonders bevorzugt von 140 kPa bis 200 kPa, ganz besonders bevorzugt von 155 kPa bis 190 kPa und insbesondere von 160 kPa bis 185 kPa;
eine Bruchdehnung von 150 % bis 500 %, mehr bevorzugt von 200 % bis 380 %, noch mehr bevorzugt von 240 % bis 340 %, insbesondere 260 % bis 320 %;
einen mechanischen Verlustfaktor von 0,1 bis 1,0, mehr bevorzugt von 0,15 bis 0,8, noch mehr bevorzugt von 0,2 bis 0,6;
eine Härte von 20 bis 70 Shore A, mehr bevorzugt von 30 bis 60 Shore A, noch mehr bevorzugt von 40 bis 50 Shore A;
eine Zellzahl (= Anzahl der Poren entlang einer Geraden pro laufendem cm) von 3 bis 40 cm⁻¹, bevorzugt 5 bis 25 cm⁻¹, mehr bevorzugt 7 bis 18 cm⁻¹, noch mehr bevorzugt 8 bis 15 cm⁻¹;
eine Rohdichte zwischen 15 und 55 kg/m³, mehr bevorzugt zwischen 20 und 35 kg/m³, noch mehr bevorzugt zwischen 22 und 30 kg/m³, insbesondere zwischen 24 und 28 kg/m³; und/oder
eine Luftdurchlässigkeit von 1000 bis 8000 l/(m²sec), mehr bevorzugt von 1500 bis 6000 l/(m²sec), noch mehr bevorzugt von 2000 bis 5000 1/(m²sec), besonders bevorzugt von 2300 bis 4000 1/(m²sec) und insbesondere von 2400 bis 3300 l/(m²sec) aufweist;
und bevorzugt erhältlich ist durch Umsetzung von einem Polyisocyanat (i), ausgewählt aus MDI, PMDI und/oder TDI mit einem (ii) Polyesterpolyol, welches bevorzugt erhältlich ist durch Umsetzung einer Dicarbonsäure mit 4 bis 8 Kohlenstoffatomen mit einen Dialkohol mit 2 bis 6 Kohlenstoffatomen, wobei das (ii) Polyesterpolyol bevorzugt ein gewichtsmittleres Molekulargewicht von 500 bis 4000 g/mol aufweist; und/oder
der offenzellige Polyurethanschaumstoff einen Aromatenanteil von 5 % bis 50 %, mehr bevorzugt von 10 % bis 45 %, insbesondere von 15 % bis 40 %, aufweist.

Auch sollen im Rahmen dieser Erfindung beliebige Kombinationen aller vorstehend angegebenen Bereiche umfasst sein. So weist ein besonders bevorzugter Schaumstoff z.B. nach dreitägiger Lagerung in Rinderserum eine Zugfestigkeit von 155 kPa und 190 kPa; eine Bruchdehnung von 260 % bis 320 %; eine Zellzahl von 8 bis 15 cm⁻¹; eine Rohdichte zwischen 24 und 28 kg/m³; und/oder eine Luftdurchlässigkeit von 2400 bis 3300 1/(m²sec) auf.

Dieser Schaumstoff ist bevorzugt erhältlich durch Umsetzung von einem Polyisocyanat (i), ausgewählt aus MDI, PMDI und/oder TDI mit einem (ii) Polyesterpolyol, welches bevorzugt erhältlich ist durch Umsetzung einer Dicarbonsäure mit 4 bis 8 Kohlenstoffatomen mit einen Dialkohol mit 2 bis 6 Kohlenstoffatomen, wobei das (ii) Polyesterpolyol bevorzugt ein gewichtsmittleres Molekulargewicht von 500 bis 4000 g/mol aufweist.

Weiterhin stellt die Erfindung ein gebrauchsfertiges Set zur UnterdruckWundbehandlung umfassend die erfindungsgemäße Vorrichtung, wobei der Polyurethanschaumstoff als Wundauflage geeignet ist und gebrauchsfertig abgepackt vorliegt.

Gegenstand der Erfindung ist somit ein gebrauchsfertiges Set zur UnterdruckWundbehandlung, umfassend
(a) Abdeckmaterial zum luftdichten Verschließen eines Wundraums, d.h. der Wunde und der Wundumgebung,
(b) gegebenenfalls ein Mittel zum Anschließen einer Unterdruckquelle, bevorzugt ein Mittel zur funktionellen Verbindung des Wundraums mit einer außerhalb des Abdeckmaterials befindlichen Unterdruckquelle, so dass ein Unterdruck im Wundraum herstellbar ist und Flüssigkeiten aus dem Wundraum absaugbar sind, und
(c) eine gebrauchsfertig abgepackte Wundauflage, enthaltend einen offenzelligen Polyurethanschaumstoff, der die vorstehend beschriebenen Eigenschaften hat, z.B. eine Zugfestigkeit nach dreitägiger Lagerung in Rinderserum, gemessen gemäß DIN 53571, zwischen 80 kPa und 300 kPa.

Die vom Set umfasste abgepackte Wundauflage (c) wird bevorzugt feuchtigkeitsdicht abgepackt. Vorzugsweise liegt die gebrauchsfertige Wundauflage in steriler Form vor, wobei die Sterilisation insbesondere durch Strahlen-Sterilisation und/oder Ethylenoxid erfolgt ist. Das Set kann weitere optionale Bestandteile wie beispielsweise Klebemittel zum Fixieren des Verbands, Abdichtmittel zur Herstellung einer luftundurchlässigen Abdichtung des Verbands, Drucksensoren, Verbindungselemente für Drucksensoren, zusätzliche Schläuche, Verbindungsstücke für Schläuche, Desinfektionsmittel, Hautpflegemittel, pharmazeutische Zubereitungen oder eine Gebrauchsanweisung enthalten. Bevorzugt enthält das erfindungsgemäße Set ferner Schere, Tupfer und/oder Pinzette, insbesondere in steriler Form. Vorzugsweise umfasst das Set weiterhin eine gebrauchsfertige Unterdruckeinheit.

Ein weiterer Gegenstand der Erfindung ist die Verwendung der vorstehend erläuterten Wundauflage (c) zur oder in der Unterdrucktherapie von Wunden. Gegenstand der Erfindung ist somit auch die Verwendung des speziellen vorstehend beschriebenen offenzelligen Polyurethanschaumstoffs zur Unterdrucktherapie von Wunden, insbesondere als Wundauflage. Insbesondere ist Gegenstand der Erfindung die Verwendung eines offenzelligen Polyurethanschaumstoffs, der eine Zugfestigkeit nach dreitägiger Lagerung in Rinderserum, gemessen gemäß DIN 53571, zwischen 80 kPa und 300 kPa aufweist, zur Unterdrucktherapie von Wunden, insbesondere als Wundauflage (c). Alle vorstehend aufgeführten Erläuterungen zu bevorzugten Ausführungsformen der Komponente (c), d.h. zu dem offenzelligen Polyurethanschaumstoff finden nicht nur für die erfindungsgemäße Vorrichtung, sondern auch für die erfindungsgemäße Verwendung Anwendung.

Besondere Vorteile durch die erfindungsgemäße Vorrichtung, durch das erfindungsgemäße Set oder die erfindungsgemäße Verwendung, beziehungsweise Anwendung, ergeben sich, wenn es sich bei den Wunden um Brandwunden, um durch mechanische Traumatisierung entstandene Wunden, um eine durch Einwirkung von Chemikalien entstandene Wunde, um eine durch eine Stoffwechselstörung verursachte Wunde, um eine durch eine Durchblutungsstörungen verursachte Wunde, oder um eine durch ein Druckgeschwür verursachte Wunde handelt, insbesondere, wenn es sich bei den genannten Wunden um chronische Wunden handelt. Des weiteren können Wunden besonders vorteilhaft behandelt werden, die durch das diabetische Fußsyndrom bedingt sind. Ferner können durch Strahlengeschwüre verursachte Wunden vorteilhaft behandelt werden.

In einer weiteren bevorzugten Ausführungsform wird die Wundauflage (c) zur Anwendung in der Unterdrucktherapie bei der Behandlung einer durch eine Hauttransplantation entstandenen Wunde bereitgestellt. Die Anwendung umfasst die Behandlung von durch Spalthaut-Transplantationen und von durch Vollhaut-Transplantationen entstandenen Wunden mittels Unterdrucktherapie. Vorteilhafte Wirkungen ergeben sich durch die Struktur des speziellen offenzelligen Polyurethanschaumstoffs, der bevorzugt eine Zugfestigkeit nach dreitägiger Lagerung in Rinderserum gemessen, gemäß DIN 53571, zwischen 80 kPa und 300 kPa aufweist, sowie durch die gleichmäßige Druckverteilung. Bei der Anwendung der Wundauflage (c) bei der Behandlung einer durch eine Hauttransplantation entstandenen Wunde kann das Transplantat ("Skin-Graft") ausreichend fixiert und gleichzeitig können schädliche Scherkräfte vermieden werden.

Die vorstehend beschriebene Wundauflage (c) kann in der Unterdrucktherapie von Druckwunden bei Patienten mit einem Body-Mass-Index (BMI = Körpermasse durch Körperlänge im Quadrat) von weniger als 18,0, insbesondere mit einem Body-Mass-Index von 14 bis 17,5 vorteilhaft verwendet werden. Dies gilt insbesondere, wenn es sich um Patienten mit einem Alter von mehr als 60 Jahren handelt. Insbesondere bei diesen Patienten zeigt sich die vorteilhafte Wirkung der erfindungsgemäßen Vorrichtung oder des erfindungsgemäßen Sets.

Ein weiterer Gegenstand der Erfindung ist ein Verfahren zur Unterdrucktherapie von Wunden, umfassend die Schritte
a) Bereitstellen einer Vorrichtung nach einem der Ansprüche 1 bis 13;
b) Anlegen des Unterdruckverbandes an der Wunde;
c) Herstellung eines Unterdruckes von 25 bis 250 mm Hg, bevorzugt von 50 bis 150 mm Hg im Wundraum für mindestens 30 Minuten und höchstens 7 Tage, bevorzugt für mindestens 1 Tag und höchstens 6 Tage.

### FIGUREN

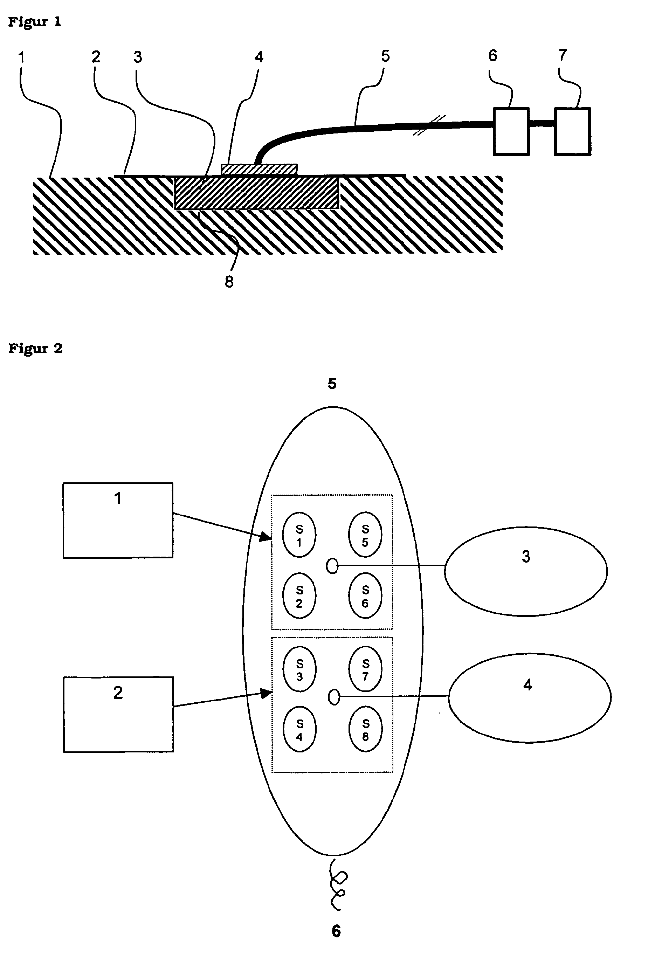

- **Figur 1:**: schematischer Aufbau der erfindungsgemäßen Vorrichtung (Seitenansicht)

| | |
|---|---|
| 1 | Wundumgebung (d.h. in der Regel unversehrte Haut) |
| 2 | Luftundurchlässiges Abdeckmaterial (a) |
| 3 | Wundauflage (c) = offenzelliger Polyurethanschaumstoff |
| 4 | Unterdruck- Anschlussstück (Port) |
| 5 | Unterdruck-Verbindungsleitung |
| 6 | Sammelgefäß |
| 7 | Unterdruckeinheit |
| 8 | Wundgrund |

Die erfindungsgemäße Vorrichtung zur Unterdrucktherapie von Wunden wird anhand von **Figur 1** näher erläutert. In Figur 1 wird der schematischen Aufbau der erfindungsgemäßen Vorrichtung in der Seitenansicht dargestellt. Die Vorrichtung umfasst ein luftundurchlässiges Abdeckmaterial (2), ein Mittel (4-5) zur funktionellen Verbindung des Wundraums mit einer außerhalb des Abdeckmaterials befindlichen Unterdruckquelle (7), sowie dem offenzelligen Schaumstoff (3). Das Abdeckmaterial (2) ist im Bereich der Wundumgebung (1), welche üblicherweise unversehrte Haut aufweist, befestigt. Die Größe des Abdeckmaterials sollte so bemessen sein, dass das Abdeckmaterial außerhalb des Wundbereichs im Bereich der Wundumgebung (1) befestigt werden kann. Das Abdeckmaterial (2) kann in verschiedenen Abmessungen und Formen vorliegen, beispielsweise kreisförmig, oval oder rechteckig. Es kann auch in einer an die Wunde angepassten, unregelmäßigen Form vorliegen. Das Abdeckmaterial (2) wird üblicherweise im Bereich der Wundumgebung (1) befestigt und luftdicht abgedichtet. Dies kann beispielsweise dadurch erfolgen, dass das Abdeckmaterial (2) einen Kleberand aufweist. Alternativ kann eine klebende Substanz entweder auf den Rand des Abdeckmaterials (2) und/oder auf die intakte Haut im Bereich der Wundumgebung aufgebracht werden. Dies hat den Vorteil, dass eine Anpassung des Abdeckmaterials an die Form und Größe der Wunde leichter möglich ist. Das Unterdruckanschlussstück (4) ist bei der hier gezeigten bevorzugten Ausführungsform auf der von der Wunde weg weisenden Außenseite des luftundurchlässigen Abdeckmaterials (2) angebracht. Um den Wundraum mit einer außerhalb des Abdeckmaterials befindlichen Unterdruckeinheit (7) funktionell zu verbinden, muss sich bei dieser Anordnung im Bereich des Unterdruckanschlussstücks (4) eine oder mehrere durch das Abdeckmaterial (2) hindurchgehende Öffnung(en) befinden.

In einer bevorzugten Ausführungsform der Erfindung umfasst die Vorrichtung zur Unterdrucktherapie von Wunden keine Wundkontaktschicht zum Einbringen zwischen Wundauflage (3) und Wundoberfläche (8).

**Figur 2** illustriert die Bestimmung von unerwünschten Fremdpartikeln in Wunden gemäß Beispiel 2.

Die Erfindung soll durch nachfolgende Beispiele veranschaulicht werden.

### Beispiel 1: Bestimmung der Zugfestigkeit

Es wurde ein erfindungsgemäßer Schaumstoff hergestellt und ein für die Unterdruckbehandlung kommerziell erhältlicher Schaumstoff als Vergleich verwendet:

Schaumstoff A (erfindungsgemäß), erhältlich durch Umsetzung von Polyesterpolyol und Isocyanat, Zugfestigkeit nach trockener Lagerung im Normklima 160 kPa, Zugfestigkeit nach dreitägiger Lagerung in Rinderserum: 170 kPa.

Schaumstoff B (Vergleich), erhältlich durch Umsetzung von Polyetherpolyol und Isocyanat, Zugfestigkeit nach trockener Lagerung im Normklima 115 kPa, Zugfestigkeit nach dreitägiger Lagerung in Rinderserum: 72 kPa.

### Beispiel 2: Bestimmung von unerwünschten Fremdpartikeln in Wunden

Jeweils 6 Schweine wurden an 8 verschiedenen Wunden mit einer Vorrichtung zur Unterdruckwundbehandlung, umfassend entweder den gemäß Beispiel 1 erfindungsgemäßen Schaumstoff A oder den Vergleichsschaumstoff B für 7 Tage behandelt. Eine systematische Darstellung der Versuchsanordnung ist in Figur 2 erläutert. Figur 2 zeigt eine Aufsicht auf einen Schweinerücken. S1 - S8 sind die mit Schaumstoff abgedeckten Wunden, 1 und 2 stellen eine Wundauflage aus den Schaumstoffen A oder B dar, die jeweils vier Wunden abdecken. 3 und 4 sind Öffnungen zur Verbindung mit dem Unterdrucksystem. 5 ist der Kopf des Schweins, 6 der Schwanz.

Nach der Behandlungszeit wurden die Wunden auf unerwünschte Schaumstoffpartikel untersucht. Es zeigte sich folgendes Ergebnis:

Erfindungsgemäßer Schaumstoff A: 0 % der Wunden enthielten Schaumstoffpartikel

Vergleichsschaumstoff B: 25 % der Wunden enthielten Schaumstoffpartikel.

### Beispiel 3: Histologische Untersuchung der inflammaorischen Aktivität in Wunden

Wie in Beispiel 2 dargestellt wurden ein erfindungsgemäßer Schaum A und ein zum Vergleich dienender Schaum B als Wundauflagen verwendet.

Die Granulationsgewebe wurden histologisch untersucht.

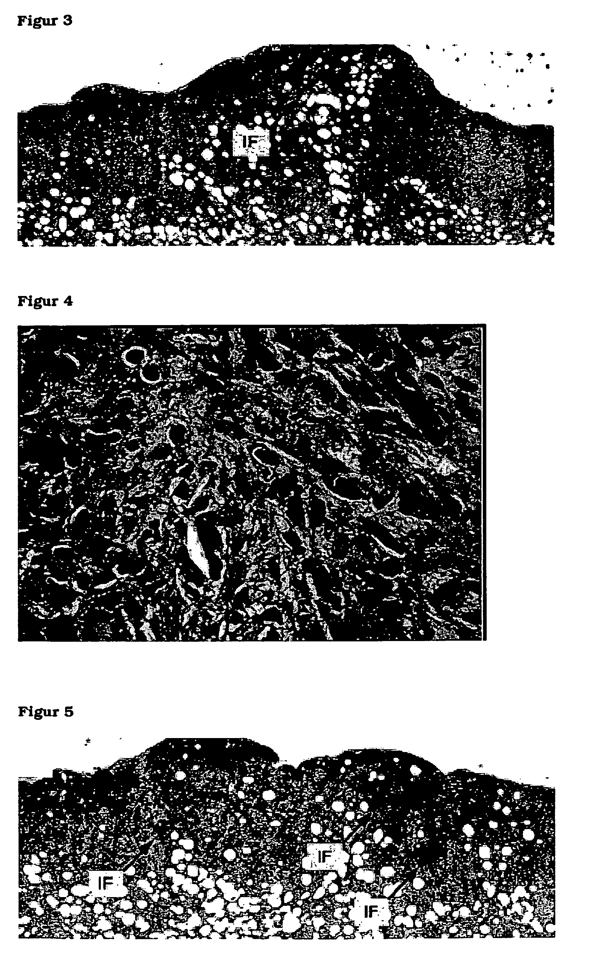

Die histologische Untersuchung einer Wunde, welche mit dem erfindungsgemäßen Schaum A behandelt wurde, zeigt nur wenige inflammatorische Foci (IF), wie in Figur 3 dargestellt. Die inflammatorischen Foci zeigen nur eine geringe Einwanderung neutrophiler Zellen, siehe Figur 4.

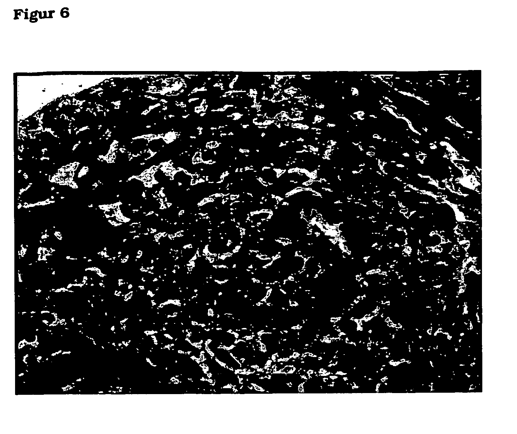

Im Gegensatz dazu existieren mehr Bereiche mit inflammatorischer Aktivität im Granulationsgewebe einer Wunde, welche mit dem zum Vergleich dienenden Schaum B behandelt wurde, wie in Figur 5 dargestellt. Die inflammatorischen Foci bestehen aus Fremdkörperriesenzellen, neutrophilen Granulozyten und lymphatischen Zellen, wie in Figur 6 dargestellt.

Folglich geht die Verwendung eines erfindungsgemäßen Schaums mit weniger inflammatorischer Aktivität einher.

## Patentansprüche

1. Vorrichtung zur Unterdrucktherapie von Wunden umfassend
(a) ein Abdeckmaterial zum luftdichten Verschließen eines Wundraums;
(b) gegebenenfalls ein Mittel zum Anschließen einer Unterdruckquelle; und
(c) einen offenzelligen Polyurethanschaumstoff als Wundauflage, erhältlich durch Umsetzung einer Mischung, umfassend die Komponenten
(i) Polyisocyanat,
(ii) Polyesterpolyol,
(iii) Treibmittel, und
(iv) Katalysator,
wobei der offenzellige Schaumstoff nach dreitägiger Lagerung in Rinderserum eine Zugfestigkeit, gemessen gemäß DIN 53571, zwischen 80 kPa und 300 kPa aufweist und wobei das (ii) Polyesterpolyol erhältlich ist durch Umsetzung einer Dicarbonsäure mit 4 bis 8 Kohlenstoffatomen mit einen Dialkohol mit 2 bis 6 Kohlenstoffatomen.

2. Vorrichtung nach Anspruch 1, wobei der offenzellige Polyurethanschaumstoff eine Zugfestigkeit nach dreitägiger Lagerung in Rinderserum, gemessen gemäß DIN 53571, zwischen 150 kPa und 220 kPa aufweist.

3. Vorrichtung nach einem der vorangehenden Ansprüche,
wobei das (i) Polyisocyanat ausgewählt ist aus MDI, PMDI und/oder TDI.

4. Vorrichtung nach einem der vorangehenden Ansprüche, wobei das (ii) Polyesterpolyol ein gewichtsmittleres Molekulargewicht von 500 bis 4000 g/mol aufweist.

5. Vorrichtung nach einem der vorangehenden Ansprüche, wobei der offenzellige Polyurethanschaumstoff eine Bruchdehnung von 250 % bis 650 %, gemessen gemäß DIN 53571, aufweist.

6. Vorrichtung nach einem der vorangehenden Ansprüche, wobei der offenzellige Polyurethanschaumstoff eine Luftdurchlässigkeit von 1000 bis 8000 1/(m²sec), gemessen gemäß DIN EN ISO 9237, aufweist.

7. Vorrichtung nach einem der vorangehenden Ansprüche, wobei offenzellige Polyurethanschaumstoff eine Rohdichte zwischen 15 und 30 kg/m³ aufweist, gemessen gemäß DIN EN ISO 845.

8. Vorrichtung nach einem der vorangehenden Ansprüche, wobei der offenzellige Polyurethanschaumstoff einen Aromatenanteil von 5 bis 50 % aufweist.

9. Vorrichtung nach einem der vorangehenden Ansprüche, wobei der offenzellige Polyurethanschaumstoff eine Härte von 20 bis 70 Shore A, gemessen gemäß DIN 53505, aufweist.

10. Vorrichtung nach einem der vorangehenden Ansprüche, wobei der offenzellige Polyurethanschaumstoff eine Zellzahl von 5 bis 25 cm⁻¹ aufweist.

11. Vorrichtung nach einem der vorangehenden Ansprüche, wobei der offenzellige Polyurethanschaumstoff viskoelastisches Verhalten zeigt.

12. Vorrichtung nach einem der vorangehenden Ansprüche, wobei das Abdeckmaterial (a) eine Wasserdampfdurchlässigkeit von 100 bis 2500 g/m² x 24 h, bestimmt gemäß DIN EN 13726-2, aufweist.

13. Verwendung eines offenzelligen Polyurethanschaumstoffs als Wundauflage zur Unterdrucktherapie von Wunden, wobei der offenzellige Polyurethanschaumstoff erhältlich ist durch Umsetzung einer Mischung, umfassend die Komponenten
(i) Polyisocyanat,
(ii) Polyesterpolyol,
(iii) Treibmittel, und
(iv) Katalysator,
und wobei der offenzellige Schaumstoff nach dreitägiger Lagerung in Rinderserum eine Zugfestigkeit, gemessen gemäß DIN 53571, zwischen 80 kPa und 300 kPa aufweist und wobei das (ii) Polyesterpolyol erhältlich ist durch Umsetzung einer Dicarbonsäure mit 4 bis 8 Kohlenstoffatomen mit einen Dialkohol mit 2 bis 6 Kohlenstoffatomen.

14. Verwendung gemäß Anspruch 13, wobei es sich um Brandwunden, um durch mechanische Traumatisierung entstandene Wunden, um durch Einwirkung von Chemikalien entstandene Wunden, um durch Stoffwechselstörungen verursachte Wunden, um durch Durchblutungsstörungen verursachte Wunden, durch ein Strahlengeschwür verursachte Wunden, durch das diabetische Fußsyndrom verursachte Wunden oder um durch Druckgeschwüre verursachte Wunden handelt.

## Claims

1. A device for negative pressure wound therapy comprising
(a) a cover material for air-tight sealing of the wound space;
(b) as applicable, a means for the connection of a negative pressure source;
and
(c) an open-cell polyurethane foam as a wound dressing, obtained by reaction of a mixture comprising the components
(i) polyisocyanate,
(ii) polyester polyol,
(iii) blowing agent, and
(iv) catalyst,
whereby the open-cell foam, after three days of storage in bovine serum, has a tensile strength, measured in accordance with DIN 53571, between 80 kPa and 300 kPa and whereby the (ii) polyester polyol is obtainable by reaction of a dicarboxylic acid with 4 to 8 carbon atoms with a dialcohol with 2 to 6 carbon atoms.

2. The device in accordance with Claim 1, whereby the open-cell polyurethane foam has a tensile strength after three days of storage in bovine serum, measured in accordance with DIN 53571, between 150 kPa and 220 kPa.

3. The device in accordance with one of the previous claims,
whereby the (i) polyisocyanate is selected from MDI, PMDI and/or TDI.

4. The device in accordance with one of the previous claims, whereby the (ii) polyester polyol has a weight average molecular weight of 500 to 4,000 g/mol.

5. The device in accordance with one of the previous claims, whereby the open-cell polyurethane foam has a ductile yield of 250% to 650%, measured in accordance with

6. The device in accordance with one of the previous claims, whereby the open-cell polyurethane foam has an air permeability of 1,000 to 8,000 1/(m²sec), measured in accordance with DIN EN ISO 9237.

7. The device in accordance with one of the previous claims, whereby the open-cell polyurethane foam has a raw density between 15 and 30 kg/m³, measured in accordance with DIN EN ISO 845.

8. The device in accordance with one of the previous claims, whereby the open-cell polyurethane foam has a proportion of aromatic compounds of 5 to 50%.

9. The device in accordance with one of the previous claims, whereby the open-cell polyurethane foam has a hardness of 20 to 70 Shore A, measured in accordance with DIN 53505.

10. The device in accordance with one of the previous claims, whereby the open-cell polyurethane foam has a cell number of 5 to 25 cm⁻¹.

11. The device in accordance with one of the previous claims, whereby the open-cell polyurethane foam displays visco-elastic behavior.

12. The device in accordance with one of the previous claims, whereby the cover material (a) has a water vapor permeability of 100 to 2,500 g/m² x 24 h, measured in accordance with DIN EN 13726-2.

13. Use of an open-cell polyurethane foam as a wound dressing for negative pressure wound therapy, whereby the open-cell polyurethane foam is obtainable by reaction of a mixture comprising the components
(i) polyisocyanate,
(ii) polyester polyol,
(iii) blowing agent, and
(iv) catalyst,
and whereby the open-cell foam, after three days of storage in bovine serum, has a tensile strength, measured in accordance with DIN 53571, between 80 kPa and 300 kPa and whereby the (ii) polyester polyol is obtainable by reaction of a dicarboxylic acid with 4 to 8 carbon atoms with a dialcohol with 2 to 6 carbon atoms.

14. Use in accordance with Claim 13, whereby the wounds are burn wounds, wounds caused by mechanical trauma, wounds caused by exposure to chemicals, wounds caused by a metabolic disorder, wounds caused by a circulatory disorder, wounds caused by radiation induced ulcer, wounds caused by diabetic foot ulcer, or wounds caused by pressure ulcers.

## Revendications

1. Dispositif permettant de soigner des plaies par application d'une pression négative, comprenant
(a) un matériau de couverture destiné à fermer un espace de plaie de manière à le rendre étanche à l'air ;
(b) le cas échéant un moyen permettant le raccordement à une source de pression négative ; et
(c) une mousse de polyuréthane à pores ouverts servant à recouvrir ladite plaie et pouvant être obtenue en faisant réagir un mélange qui comprend les composants suivants
(i) polyisocyanate,
(ii) polyesterpolyol,
(iii) agent gonflant, et
(iv) catalyseur,
ladite mousse à pores ouverts présentant, suite à un stockage dans du sérum bovin pendant trois jours, une résistance à la traction, mesurée selon la norme DIN 53571, comprise entre 80 kPa et 300 kPa et le (ii) polyesterpolyol pouvant être obtenu en faisant réagir un acide dicarboxylique renfermant 4 à 8 atomes de carbone avec un dialcool renfermant 2 à 6 atomes de carbone.

2. Dispositif selon la revendication 1, ladite mousse de polyuréthane à pores ouverts présentant, suite à un stockage dans du sérum bovin pendant trois jours, une résistance à la traction, mesurée selon la norme DIN 53571, comprise entre 150 kPa et 220 kPa.

3. Dispositif selon l'une des revendications précédentes,
le (i) polyisocyanate étant choisi parmi le MDI, le PMDI et/ou le TDI.

4. Dispositif selon l'une des revendications précédentes, le (ii) polyesterpolyol présentant une masse moléculaire moyenne en poids comprise entre 500 et 4000 g/mol.

5. Dispositif selon l'une des revendications précédentes, ladite mousse de polyuréthane à pores ouverts présentant un allongement à la rupture, mesuré selon la norme DIN 53571, compris entre 250 % et 650 %.

6. Dispositif selon l'une des revendications précédentes, ladite mousse de polyuréthane à pores ouverts présentant une perméabilité à l'air, mesurée selon la norme DIN EN ISO 9237, comprise entre 1000 et 8000 l/(m²sec).

7. Dispositif selon l'une des revendications précédentes, ladite mousse de polyuréthane à pores ouverts présentant une densité volumétrique, mesurée selon la norme DIN EN ISO 845, comprise entre 15 et 30 kg/m³.

8. Dispositif selon l'une des revendications précédentes, ladite mousse de polyuréthane à pores ouverts présentant une proportion en composés aromatiques comprise entre 5 et 50 %.

9. Dispositif selon l'une des revendications précédentes, ladite mousse de polyuréthane à pores ouverts présentant une dureté, mesurée selon la norme DIN 53505, comprise entre 20 et 70 Shore A.

10. Dispositif selon l'une des revendications précédentes, ladite mousse de polyuréthane à pores ouverts présentant un nombre de pores compris entre 5 et 25 cm⁻¹.

11. Dispositif selon l'une des revendications précédentes, ladite mousse de polyuréthane à pores ouverts présentant un comportement viscoélastique.

12. Dispositif selon l'une des revendications précédentes, le matériau de couverture (a) présentant une perméabilité à la vapeur d'eau, déterminée selon la norme DIN EN 13726-2, comprise entre 100 et 2500 g/m² x 24 h.

13. Utilisation d'une mousse de polyuréthane à pores ouverts pour recouvrir des plaies, lesdites plaies étant soignées par application d'une pression négative, ladite mousse de polyuréthane à pores ouverts pouvant être obtenue en faisant réagir un mélange qui comprend les composants suivants
(i) polyisocyanate,
(ii) polyesterpolyol,
(iii) agent gonflant, et
(iv) catalyseur,
et ladite mousse à pores ouverts présentant, suite à un stockage dans du sérum bovin pendant trois jours, une résistance à la traction, mesurée selon la norme DIN 53571, comprise entre 80 kPa et 300 kPa et le (ii) polyesterpolyol pouvant être obtenu en faisant réagir un acide dicarboxylique renfermant 4 à 8 atomes de carbone avec un dialcool renfermant 2 à 6 atomes de carbone.

14. Utilisation selon la revendication 13, s'agissant de brûlures, de plaies provoquées par un trauma mécanique, de plaies provoquées par l'action de produits chimiques, de plaies provoquées par des perturbations du métabolisme, de plaies provoquées par des troubles de la circulation sanguine, de plaies provoquées par un ulcère résultant d'une irradiation, de plaies provoquées le pied diabétique ou de plaies provoquées par des ulcères résultant d'une pression.
